(19) Europäisches Patentamt
European Patent Office
Office européen des brevets

(11) Veröffentlichungsnummer: **0 253 275 B1**

(12) **EUROPÄISCHE PATENTSCHRIFT**

(45) Veröffentlichungstag der Patentschrift: **06.03.91**

(51) Int. Cl.[5]: **C07C 213/00**, C07C 215/06, C07C 217/04, A61K 7/075

(21) Anmeldenummer: **87109763.0**

(22) Anmeldetag: **07.07.87**

# (54) Neue quartäre Ammoniumverbindungen und deren Verwendung.

(30) Priorität: **14.07.86 DE 3623730**

(43) Veröffentlichungstag der Anmeldung: **20.01.88 Patentblatt 88/03**

(45) Bekanntmachung des Hinweises auf die Patenterteilung: **06.03.91 Patentblatt 91/10**

(84) Benannte Vertragsstaaten: **AT BE CH DE ES FR GB IT LI NL SE**

(56) Entgegenhaltungen:
**DE-A- 3 116 087**
**DE-A- 3 345 156**
**DE-A- 3 442 175**
**GB-A- 1 087 413**

(73) Patentinhaber: **Henkel Kommanditgesellschaft auf Aktien**
**Postfach 1100 Henkelstrasse 67**
**W-4000 Düsseldorf-Holthausen(DE)**

(72) Erfinder: **Lange, Fritz, Dr.**
**Baderweg 82**
**W-4300 Essen(DE)**
Erfinder: **Rutzen, Horst, Dr.**
**Falkenweg 12**
**W-4018 Langenfeld(DE)**
Erfinder: **Busch, Peter, Dr.**
**Gottfried-August-Bürger-Strasse 10**
**W-4006 Erkrath(DE)**
Erfinder: **Thiele, Klaus**
**Rügenweg 5**
**W-4018 Langenfeld(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

**Beschreibung**

Gegenstand der Erfindung sind neue quartäre Ammoniumverbindungen und deren Verwendung als haarkonditionierende und die Kämmbarkeit verbessernde Zusätze in haarkosmetischen Mitteln.

Kationische Tenside vom Typ der quartären Ammoniumverbindungen werden in der Haarkosmetik als avivierende und konditionierende Wirkstoffe zur Verbesserung der Kämmbarkeit, der Fülle und des Griffs der Haare und zur Senkung der statischen Aufladbarkeit der Haare eingesetzt. Die Produkte werden meist in Haarnachbehandlungsmitteln verwendet, die nach der Haarwäsche dem Haar wieder günstige haarkosmetische Eigenschaften verleihen sollen. Sie eignen sich aber wenig als Zusatzmittel zu Haarwaschmitteln, um gleichzeitig mit der Wäsche einen gewissen konditionierenden Effekt zu erzielen, da die meisten bekannten quartären Ammoniumverbindungen mit den in Shampoos gebräuchlichen schaumstarken anionischen Tensiden, in den für eine ausreichende konditionierende Wirkung erforderlichen Konzentrationen nicht verträglich sind, sondern schwer wasserlösliche und kosmetisch unwirksame Niederschläge bilden. Quartäre Ammoniumverbindungen, die mit Aniontensiden besser verträglich sind, weisen meist eine unbefriedigende konditionierende Wirkung auf.

Wasserlösliche kationische Polymere sind zwar meist mit anionischen Tensiden verträglich, haben aber andere Nachteile, z.B. Akkumulation auf dem Haar nach mehrmaliger Behandlung und die unzureichende Verminderung der statischen Aufladbarkeit des trockenen Haars.

Es bestand daher die Aufgabe, quartäre Ammoniumverbindungen zu finden, die starke avivierende und konditionierende Eigenschaften auf menschlichem Haar besitzen und die auch in Haarwaschmittel (Shampoos) auf Basis schaumstarker anionischer Tenside in Konzentrationen eingearbeitet werden können, die für eine befriedigende kosmetische Wirkung ausreichend sind ohne daß Trübungen und Ausfällungen auftreten.

Es wurden neue quartäre Ammoniumverbindungen gefunden, welche die vorstehend genannten Anforderungen in hohem Maße erfüllen. Die neuen quartären Ammoniumverbindungen sind gekennzeichnet durch die Formel I

I) 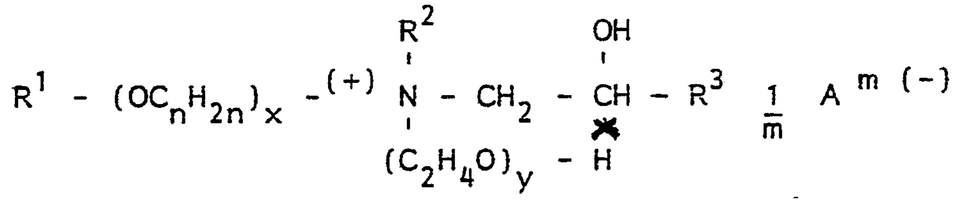

in der $R^1$ eine Alkylgruppe mit 8 bis 22 C-Atomen, $R^2$ eine Alkylgruppe mit 1 bis 4 C-Atomen oder eine Gruppe $-(C_2H_4O)_z-H$ und $R^3$ eine Alkylgruppe mit 6 bis 20 C-Atomen, x = o oder eine Zahl von 1 bis 10, y und z Zahlen von 1 bis 10, n = 2 oder 3, A das Anion einer anorganischen Säure oder einer organischen Carbon-oder Sulfonsäure mit 1 bis 18 C-Atomen und m dessen Wertigkeit ist.

Die Herstellung der neuen quartären Ammoniumverbindungen erfolgt ausgehend von tertiären Etheraminen, die in Form ihrer Salze beziehungsweise in stöchiometrischen Mengen mit einer zur Salzbildung geeigneten anorganischen Säure oder einer organischen Carbon- oder Sulfonsäure mit bis zu 18 C-Atomen eingesetzt werden. Die Salze der tertiären Etheramine entsprechen der Formel II

II) 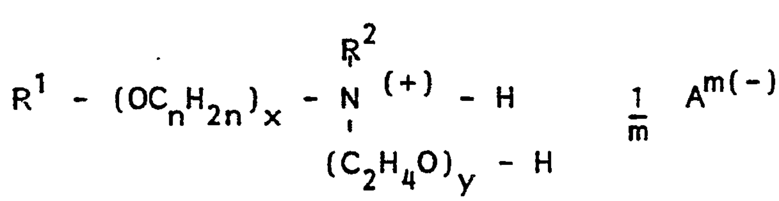

Diese werden mit 1 Mol eines alpha-Epoxids der Formel

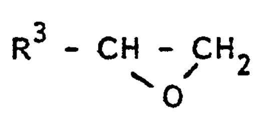

zur Umsetzung gebracht. $R^1$, $R^2$, $R^3$, n, x, y, A und m haben dabei die für Formel I angegebene Bedeutung.

Die den Salzen der Formel II zugrundeliegenden Etheramine sind literaturbekannt, z.B. aus GB-A-1.087.413 und aus EP-A-102 140. Nach dem dort beschriebenen Herstellverfahren werden solche Etheramine ausgehend von primären und sekundären Aminen durch Alkylierung am Stickstoffatom, z.B. mit Alkylpolyglycolethersulfaten hergestellt. Ein weiteres Verfahren, das Gegenstand der deutschen Patentanmeldung P 35 04 242.7 ist, geht aus von tertiären Alkanolaminen und führt durch Alkylierung an einer Hydroxylgruppe, z.B. mit Alkylsulfaten oder Alkylpolyglycolethersulfaten zu tertiären Etheraminen. Die Überführung der tertiären Etheramine in die Salze der Formel II erfolgt z.B. durch Zugabe von Mineralsäuren wie z.B. konzentrierter Salzsäure, konzentrierter Bromwasserstoffsäure, Schwefelsäure, Phosphorsäure oder Borsäure oder organischer Säuren wie z.B. Ameisensäure, Essigsäure, Benzoesäure, Benzolsulfonsäure, p-Toluolsulfonsäure, Isononansäure oder Laurinsäure. Es können auch mehrbasische organische Säuren wie z.B. Citronensäure, Äpfelsäure oder Weinsäure, Maleinsäure oder Bernsteinsäure verwendet werden.

Die Umsetzung der Salze der tertiären Etheramine der Formel II mit alpha-Epoxiden der Formel

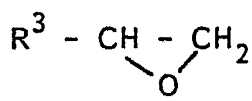

erfolgt nach an sich bekannten Verfahren. Ein besonders geeignetes Verfahren ist z.B. in DE-OS 31 16 087 angegeben. Nach den dort beschriebenen Verfahren wird die Umsetzung in Gegenwart einer quartären Ammoniumverbindung als Phasentransferkatalysator durchgeführt.

Verbindungen der Formel II, in welchen y oder z Werte von mehr als 1 aufweisen, können dadurch erhalten werden, daß man Etheramine der Formel

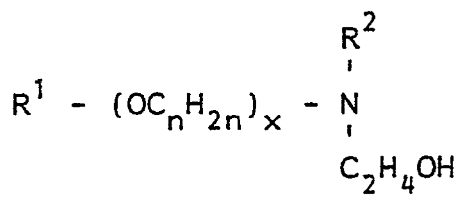

in der $R^2$ gegebenenfalls eine Gruppe $-C_2H_4OH$ ist, mit (y - 1) + (z - 1) Mol Ethylenoxid nach dem dafür bekannten Verfahren umsetzt. Auf diese Weise läßt sich die Hydrophilie der erfindungsgemäßen quartären Ammoniumverbindungen beliebig erhöhen.

Die erfindungsgemäßen quartären Ammoniumverbindungen haben oberflächenaktive Eigenschaften und wie alle quartären Ammoniumtenside sind sie substativ, d.h. sie ziehen auf Faseroberflächen auf und zeigen dort antistatische und avivierende Eigenschaften. Darüber hinaus lassen sie sich in wässrigen Tensidlösungen klar solubilisieren. Überraschenderweise gelingt es, die erfindungsgemäßen quartären Ammoniumverbindungen auch in wässrigen Lösungen anionischer Tenside klar oder nur schwach opal zu solubilisieren ohne daß es zur Bildung schwerlöslicher Elektroneutralsalze kommt. Diese Eigenschaft macht die erfindungsgemäßen quartären Ammoniumverbindungen besonders geeignet für die Verwendung als konditionierende und avivierende Wirkstoffe in haarkosmetischen Mitteln.

Unter haarkosmetischen Mitteln werden alle Zubereitungen auf wässriger und wässrig-alkoholischer Basis verstanden, die zur pflegenden und dekorativen Behandlung des menschlichen Haares geeignet sind wie z.B. Haarwässer, Haarlotionen, Haarkurmittel, Haarfestiger, Haarfärbemittel, Haarwellmittel, Haarnachbehandlungsmittel, Haarspülmittel und Haarwaschmittel (Shampoo). Bevorzugt sind erfindungsgemäße quartäre Ammoniumverbindungen der Formel I, worin x eine Zahl von 2 bis 10, y = 1, $R^2$ eine 2-Hydroxyethylgruppe und A ein Chloridanion ist.

Eine besonders bevorzugte Ausführungsform der Erfindung sind wässrige Shampoos mit einem Gehalt von 5 bis 25 Gewichtsprozent anionischer Sulfat und/oder Sulfonattenside und 0,1 bis 5 Gewichtsprozent quartärer Ammoniumverbindungen der Formel I. Unter anionischen Sulfat- oder Sulfonattensiden werden dabei Alkali- und/oder Mono-, Di- oder Trialkanolaminsalze mit 2- oder 3 C-Atomen in der Alkanolgruppe, von oberflächenaktiven Stoffen verstanden, die im Molekül eine ganz oder vorwiegend lineare Alkylgruppe mit 10 bis 16 C-Atomen und eine anionische $-SO_3^{(-)}$ oder $OSO_3^{(-)}$ Gruppe enthalten. Bevorzugte Beispiele solcher Sulfat- oder Sulfonattenside sind Fettalkoholsulfate, Fettalkoholpolyglycolethersulfate (mit 1 bis 12 Glycolethergruppen), sekundäre Alkansulfonate und Alphaolefinsulfonate.

Die folgenden Beispiele sollen den Gegenstand der Erfindung näher erläutern ohne ihn hierauf zu beschränken.

Beispiele

1. $C_{12}$-$C_{14}$ (70:30)-Alkyl-poly (2) oxyethyl-di (2-hydroxyethyl)-2-hydroxydodecyl-ammoniumchlorid

189 g (0,5 Mol) $C_{12}$-$C_{14}$-(70:30)-Alkyl-poly (2) oxyethyl-di(2-hydroxyethyl)-amin (Aminzahl 161, hergestellt aus $C_{12}C_{14}$-Alkyl + 2 EO-sulfat, Na-Salz und Diethanolamin) wurde mit 331 ml (0,4 Mol) konzentrierte Salzsäure und 94 g (0,5 Mol) 1-Epoxydodecan unter Rühren auf 95 °C erwärmt. Nach 6 Stunden war eine klare, braune Lösung entstanden, wobei der Reaktionsverlauf durch Bestimmung der Epoxidzahl verfolgt wurde. Es wurden dann weitere 8,3 ml (0,1 Mol) konzentrierte Salzsäure zugegeben und noch weitere 30 Minuten bei 95 °C gerührt. Dann wurde am Rotationsverdampfer das Wasser abgezogen. Es wurde eine braune viskose

| | | |
|---|---|---|
| Epoxidzahl: | 0,3 | (Gew.-% Epoxid-Sauerstoff) |
| Hydroxylzahl: | 268,8 | (nach DGF-C-V 17a) |
| Kationtensid: | 163,3 | mval/100 g (Bestimmt nach der Zweiphasentitrations-Methode - DIN ISO 2871) |

Substanz mit folgenden Kenndaten erhalten:

2. Kokos ($C_{12}C_{18}$)-alkyl-poly(6)oxyethyl-di-(2hydroxyethyl)-2-hydroxydodecyl-ammoniumchlorid

1000 g (1,48 Mol) Kokos ($C_{12}$-$C_{18}$)-alkyl-poly (6) oxyethyl-di-(2-hydroxyethyl)amin (Aminzahl 83, hergestellt aus Kokos ($C_{12}$-$C_{18}$)-alkyl + 6 EO-sulfat, Na-Salz und Diethanolamin) 278,1 g (1,48 Mol) 1-Epoxydodecan und 98 ml konzentrierter Salzsäure wurden unter Rühren auf 95 °C erwärmt. Nach 6 Stunden wurden weitere 24,5 ml konzentrierter Salzsäure zugesetzt und noch 30 Minuten bei 95 °C gerührt. Dann wurde der Ansatz am Rotationsverdampfer getrocknet. Es wurde ein klares, braunes, viskoses Produkt mit folgenden Kenndaten erhalten:

| | | |
|---|---|---|
| Epoxidzahl: | 0,3 | (Gew.-% Epoxid-Sauerstoff) |
| Hydroxylzahl: | 198 | |
| Kationtensid: | 116 | mval/100 g (Bestimmung nach der Zweiphasentitrations-Methode) - DIN ISO 2871) |

3. $C_{12}C_{14}$ (50:50)-alkyl-poly (4,6) oxyethyl-di-(2-hydroxyethyl)-2-Hydroxydodecyl-ammonium-isononanoat.

3.1 271 kg (6,775 . $10^3$ Mol) Natriumhydroxid (Schuppen), 3720 kg (5,661 . $10^3$ Mol) Fettalkohol $C_{12}$-$C_{14}$ (50:50)-3,6 EO-sulfat, Na-Salz (Schwefelsäurehalbestersalz des Anlagerungsproduktes von 3,6 Mol Ethylenoxid an ein Fettalkoholgemisch aus 50 Gewichtsprozent Laurylalkohol und 50 Gewichtsprozent Myristylalkohol, 70 %ige wässrige Lösung) und 1013 kg (6,79 . 10 Mol) Triethanolamin wurden in einem Reaktionsbehälter gemischt und gemeinsam unter allmählichem Aufheizen innerhalb 8 Stunden auf 100 °C und innerhalb weiterer 4 Stunden auf 160 °C vorsichtig unter Rühren im Wasserstrahlvakuum entwässert. Dann wurde das Reaktionsgemisch weitere 8 Stunden auf 160 °C erhitzt. Nach dem Abkühlen auf 20 °C wurde der Ansatz einmal mit Wasser und zweimal mit gesättigter Kochsalzlösung gewaschen, dann durch Aufheizen auf 90 °C im Wasserstrahlvakuum getrocknet. Ausgefallenes Natriumchlorid wurde abfiltriert. Es wurde ein gelbes Öl mit einer Aminzahl von 103 erhalten.

3.2 590 g des Reaktionsproduktes nach 3.1 wurden mit 188 g 1-Epoxydodecan und 128 g Isononansäure in einem Reaktionsgefäß gemischt, auf 95 °C erwärmt und 4 Stunden bei dieser Temperatur gerührt. Dann wurden weitere 32 g Isononan säure zugesetzt und die Reaktion noch 1/2 Stunde fortgeführt.

Es wurde ein viskoses gelbbraunes Produkt erhalten.

Epoxidzahl: 0,3 (Gew.-% Epoxid-Sauerstoff)

4. Haarkosmetische Prüfung

Die erfindungsgemäßen quartären Ammoniumverbindungen gemäß Beispiel 1 und 2 wurden in Rezepturen 4.2 und 4.3 gemäß Tabelle I eingearbeitet und gegenüber einem Standard-Shampoo (Rezeptur 4,1) ohne quartäre Ammoniumverbindung sowie einem Vergleichsshampoo mit Cetyltrimethylammoniumchlorid (Rezeptur 4.4) anwendungstechnisch auf die Naßkämmbarkeit verbessernde Wirkung geprüft. Messung der Naßkämmbarkeit (Laborprüfung)

Es wurden standardisierte, unter definierten Bedingungen durch Blondierung und Kaltwelle vorgeschädigte Haarsträhnen verwendet. Diese wurden mit den in Tabelle 1 angegebenen Shampoos in einer Menge von 1 g pro g Haar in handwarmem Wasser shampooniert und mit klarem Wasser nachgespült. Die Messung der Naßkämmbarkeit erfolgte durch eine Messung des Kämmwiderstandes, d.h. der Kraft, die erforderlich ist, um einen Kamm durch ein Haarbüschel zu ziehen. Dabei wurde eine modifizierte Zug-Prüfmaschine des Typs 1402 der Fa. Zwick (Einsingen bei Ulm/Donau) verwendet. Die Prüfungsanordnung ist beschrieben in "Riechstoffe, Aromen, Kosmetika" Nr. 12, (1977), Seite 325, Spalte 2 und 3.

Um die Fehler möglichst gering zu halten, wurden 15-fache Bestimmungen des Kämmwiderstandes mit jedem der zu prüfen den Shampoos durchgeführt und die Mittelwerte gebildet. Die gemessenen Kämmwiderstandsmittelwerte wurden in Prozent des Standards angegeben. Der Standard wurde bestimmt nach Shampoonieren mit dem Shampoo 4.1 ohne quartäre Ammoniumverbindung und Nachspülen mit klarem Wasser.

Die Ergebnisse der Prüfung sind der Tabelle zu entnehmen:

## Tabelle

| Prüfshampoos | 4.1 | 4.2 | 4.3 | 4.4 |
|---|---|---|---|---|
| Fettalkohol ($C_{12}C_{14}$) + 2 EO-sulfat, Na-Salz (28 %ig) | 50 | 50 | 50 | 50 |
| QAV Beispiel 1 | - | 2 | - | - |
| QAV Beispiel 2 | - | - | 2 | - |
| Cetyltrimethylammonium-chlorid | - | - | - | 2 |
| Wasser (vollentsalzt) | 50 | 48 | 48 | 48 |
| Aussehen bei 20 °C | klar | klar | klar | trüb (Bodensatz) |
| Naßkämmbarkeit (% Kämmwiderstand) | 100 | 78 | 74 | 88 |

**Ansprüche**

**1.** Quartäre Ammoniumverbindungen der Formel I

$$(I)\quad R^1 - (OC_nH_{2n})_x - {}^{(+)}\underset{(C_2H_4O)_y-H}{\overset{R^2}{\overset{|}{\underset{|}{N}}}} - CH_2 - \overset{OH}{\overset{|}{CH}} - R^3 \quad \frac{1}{m}\; A^{m\,(-)}$$

in der $R^1$ eine Alkylgruppe mit 8 bis 22 C-Atomen, $R^2$ eine Alkylgruppe mit 1 bis 4 C-Atomen oder eine Gruppe $-(C_2H_4O)_z-H$ und $R^3$ eine Alkylgruppe mit 6 bis 20 C-Atomen, x = 0 oder eine Zahl von 1 bis 10, y und z Zahlen von 1 bis 10, n = 2 oder 3, A das Anion einer anorganischen Säure oder einer

organischen Carbon- oder Sulfonsäure mit 1 bis 18 C-Atomen und m dessen Wertigkeit ist.

2. Quartäre Ammoniumverbindungen gemäß Anspruch 1, dadurch gekennzeichnet, daß x eine Zahl von 2 bis 10, y = 1, $R^2$ eine 2-Hydroxylgruppe und A ein Chloridanion ist.

3. Verfahren zur Herstellung quartärer Ammoniumverbindungen der Formel I gemäß Anspruch 1 oder 2, dadurch gekennzeichnet, daß man tertiäre Etheramine in Form ihrer Salze der Formel II

II) 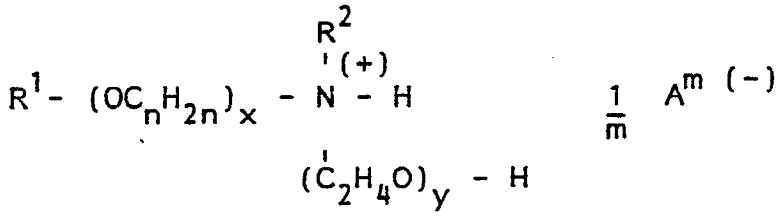

mit 1 Mol eines alpha-Epoxids der Formel

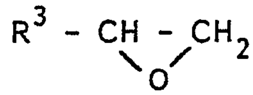

zur Umsetzung bringt.

4. Verwendung der quartären Ammoniumverbindungen der Formel I nach Anspruch 1 oder 2 als konditionierende und avivierende Wirkstoffe in haarkosmetischen Zubereitungen.

## Claims

1. Quaternary ammonium compounds corresponding to the following formula

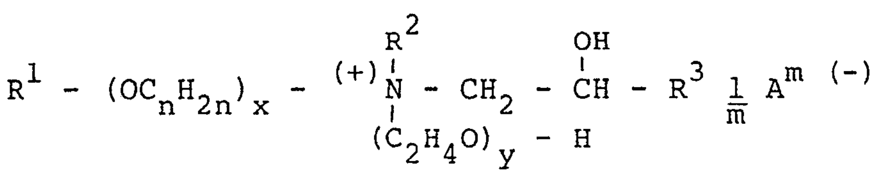 (I)

in which $R^1$ is a $C_8$-$C_{22}$ alky1 group, $R^2$ is a $C_1$-$C_4$ alkyl group or a group - $(C_2H_4O)_z$-H and $R^3$ is a $C_6$-$C_{20}$ alkyl group, x = 0 or a number of from 1 to 10, y and z are numbers of from 1 to 10, n = 2 or 3, A is the anion of an inorganic acid or of an organic carboxylic or sulfonic acid containing from 1 to 18 C-atoms and m is its valency.

2. Quaternary ammonium compounds as claimed in claim 1, characterized in that x is a number of from 2 to 10, y = 1, $R^2$ is a 2-hydroxyl group and A is a chloride anion.

3. A process for producing the quaternary ammonium compounds of formula I claimed in claim 1 or 2, characterized in that the tertiary ether amines are reacted in the form of their salts corresponding to the following formula

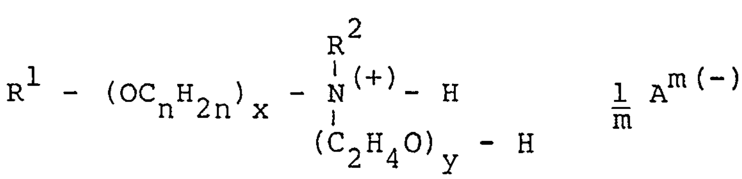 (II)

with 1 mol of an α-epoxide corresponding to the formula

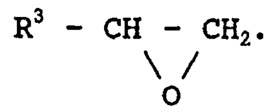

4. The use of the guaternary ammonium compounds of formula I claimed in claim 1 or 2 as conditioning and revitalizing agents in hair-cosmetic preparations.

## Revendications

1. Composés d'ammonium quaternaire de formule I

I) 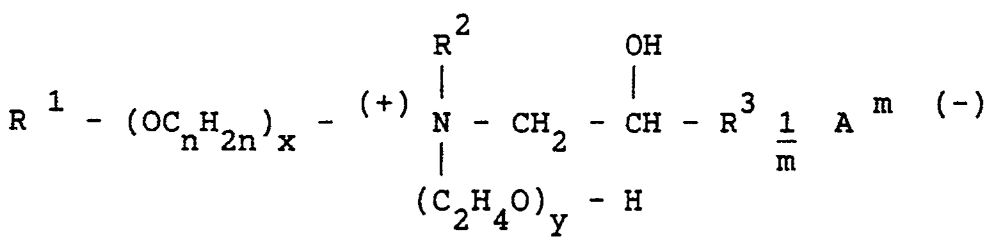

dans laquelle $R^1$ est un radical alcoyle ayant de 8 à 22 atomes de carbone,
$R^2$ est un radical de 1 à 4 atomes de carbone ou un groupe $-(C_2H_40)_z-H$,
et $R^3$ est un radical alcoyle ayant de 6 à 20 atomes de carbone,
x = 0 ou un nombre allant de 1 à 10,
y et z sont des nombres allant de 1 à 10,
n = 2 ou 3,
A est l'anion d'un acide minéral ou d'un acide organique carboxylique ou sulfonique ayant de 1 à 18 atomes de carbone,
et n est sa valence.

2. Composés d'ammonium quaternaire selon la revendication 1 caractérisés en ce que x est un nombre allant de 2 à 10, y = 1, $R^2$ est un groupe 2-hydroxylé et A est un anion chlorure.

3. Procédé d'obtention de composés d'ammonium quaternaire de formule I selon les revendications 1 ou 2n caractérisé en ce que l'on fait entrer en réaction une éthéramine tertiaire sous la forme d'un sel de formule II

II) 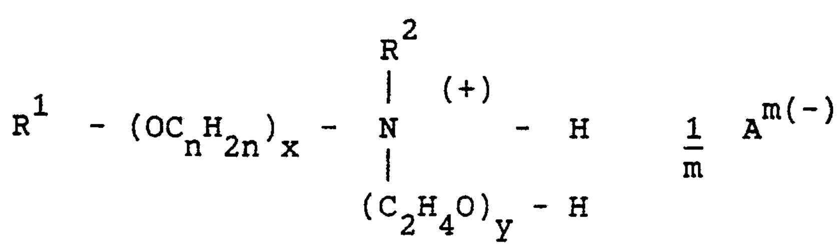

avec 1 mol d'un époxyde α de formule

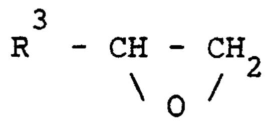

4. Utilisation des composés d'ammonium quaternaire de formule I selon les revendications 1 ou 2 comme principes actifs pour la mise en forme et pour l'avivage dans des compositions cosmétiques capillaires.